# EUROPEAN PATENT SPECIFICATION

(11) **EP 1 812 083 B1**
(45) Date of publication and mention of the grant of the patent: **06.08.2008**
(21) Application number: 06765585.2
(22) Date of filing: 23.06.2006
(51) Int. Cl.: A61L 2/00, A61L 2/07, A61L 2/18, A61B 19/00

(54) **METHOD FOR SANITISING, STERILISING AND PACKAGING OF SETS OF SURGICAL INSTRUMENTS**
VERFAHREN ZUM ENTKEIMEN, STERILISIEREN UND VERPACKEN VON SÄTZEN VON CHIRURGISCHEN INSTRUMENTEN
PROCEDE DE PREPARATION D'UN ENSEMBLE PROCEDURAL D'OUTILS CHIRURGICAUX STERILISES POUR SALLES D'OPERATION, AVEC TRAITEMENT IDENTIFIABLE D'ASSAINISSEMENT, ENTRETIEN, EMBALLAGE ET STERILISATION

(30) Priority: 27.06.2005 IT MI20051209
(43) Date of publication of application: 01.08.2007
(60) Divisional application: 07014489.4
(73) Proprietor: Steritalia S.p.A., 06019 Umbertide (PG) (IT)
(72) Inventor: STACCINI, Umbro, c/o TECNOSANIMED S.r.l., I-06015 Pierantonio (PG) (IT); ANZANELLO, Elizabetta M., c/o TECNOSANIMED S.r.l., I-06015 Pierantonio (IT)
(74) Representative: Marietti, Andrea
(86) International application number: PCT/IB2006/001711
(87) International publication number: WO 2007/000639

(56) References cited:
- WO-A-20/06078197
- DE-A1- 1 930 731
- DE-A1- 2 932 565
- US-A- 5 511 594

## Description

### Field Of the invention

The present invention relates to a method for the arrangement of a sterilized procedural set of surgical instruments (or tools) for service to operating rooms, with traceable treatment of sanitizing, maintenance, packaging, sterilization and movement.

### Background Art

It is known that in the common use at the end of the surgeries carried out inside operating rooms, the instruments used are usually subjected to a first decontamination treatment by immersion in special tanks containing disinfectant solutions and, subsequently, treated for their manual cleaning by designated operators with tools such as hard or metal brushes or by using specific machines such as surgical instrument washing machines and similar apparatus.

At the end of the cleaning, the surgical instruments already dried are suitably packaged, for instance, in sheets of paper/polypropylene or metal containers or other type and so sent for sterilization treatment. At the end of the sterilization process said instruments or set of surgical instruments are stored in a suitable environment (or room), free from humidity and dust, in order to be subsequently re-used.

It is also known that surgical instruments used for surgeries are usually owned by the Health Agencies, although they are selected for acquisition by the surgeon who performs that particular type of surgery, in as much as said instruments are thought by the surgeon to be more proper or more manageable or simpler to use for that type of surgery.

In general said surgical instruments are organized in personalized kits or they are individually packaged, depending on the different types of surgery, without guaranteeing to the operators real trackability of the surgical instruments and without a correct reconstruction of the procedures of sanitizing and sterilization being possible.

US5511594 discloses a modular pharmacy and a modular pharmacy system and process for the admixture of intravenous drugs and total parenteral nutrition solution which includes automatic daily computer download of hospital prescriptions and the daily compounding under controlled systematic aseptic conditions including individually controlled environments at each work station and following systematically controlled process steps.

DE 1930731 A1 discloses a central disinfection system for hospital beds linen. The beds, including the bed linen are cleaned and fully equipped with fresh linen in a non-sterile room, the prepared beds are then taken into another room where they are disinfected and, finally, the disinfected, equipped beds are removed to a clean room for storage or transportation back to the wards.

WO2006/078197 discloses a sterilization plant for the sterilization of surgical instruments, the plant comprising at least one original building having at least one first process line that extends, at least partly, essentially parallel to a first one of the essentially limiting external walls of said original building, the sterilization plant further comprising at least one extension module located next to and, at least partly, essentially parallel to said first external wall (2).

DE2932565 discloses process for cleaning surgical instruments inside an autoclave.

In the case in which trackability procedures are adopted, they for the most part require sanitary operators to compile special cords by hand or on a computer which are then affixed on the package to indicate the content for every package; this leads, inevitably, to unpredictable inaccuracies and errors imputable to inexperience, insufficient personal abilities or frequently pressure to proceed urgently with repackaging of the Instruments. Moreover, the surgical set to: be used changes with every surgery fixed according to the daily schedule, making it even more onerous for the personnel assigned to the withdrawal and the arrangement of the instruments in the special packaging systems for transport to the operating room.

The lack of special trackability procedures by electronic and computer means with automatic recognition of the content of the sterilized packages, can involve a multiplicity of problems, such as, for instance, loss of instruments, exchange of instruments used for different surgeries, with consequent time wasted searching for and/of substituting them where necessary. Risks associated with such errors, in the worst of the hypotheses, include discomfort for the patient being forced to undergo the surgery with a set of instruments that is incomplete or not suited to that type of surgery. Furthermore, in extreme cases, but not so rare, there are episodes in which a surgical instrument Is forgotten inside the body of the patient with serious, at times fatal, consequences for the patient himself.

Inside hospitals the operations of sanitizing, disinfection and sterilization are carried out in areas not subjected to climatic environmental and aeraulic controls such as, for instance, temperature, humidity, pressure and speed of interchange of air. There is usually no monitoring of airborne micro-bacteria or particles dispersed in the air and the layout of said environments is based as a rule on the pre-existing plant inside the hospital and are not planned to limit the diffusion of contaminating substances from one environment to another with successive steps of the treatment, nor is everything done to guarantee effective isolation when passing from one environment to another.

This necessity is felt even more since products not correctly sanitized can jeopardize the effectiveness of the sterilization: they then have to undergo a further process of cleaning before being returned for sterilization with further loss of time and increase in costs. Furthermore, inside the environments in which said treatment of sanitizing, disinfection and sterilization is carried out it is often necessary to maintain or substitute broken or worn out instruments, which requires the use of particular equipment or special environment in which to put aside the substitute instruments.

A need is therefore felt, within the hospital sector, to set up a method that allows to arrange a sterilized procedural sets of surgical instruments for service to operating rooms and that the cyclical treatment of sanitizing, maintenance, sterilization of the same and their related movement can happen together in complete safety with the possibility of tracking the surgical instruments used in an automatic way for every surgery and for every surgeon.

### Summary Of the invention

These purposes are all achieved by the method according to claim 1 related to the arrangement of sterilized procedural sets of surgical instruments for service to the operating rooms with cyclical and traceable operations of sanitizing, maintenance, sterilization and movement. It permits to identify a set of surgical instruments for every type of surgery, to furnish a graphic reproduction for the recognition of the instruments, to arrange one or more sets of surgical instruments for every type of surgery, in which every set is ordered in special metal grill-racks prepared in a closed container, sterilized in autoclave and equipped with a cover for evacuation. The sterilized set is then protected by a plastic film on which is affixed a photographic reproduction of the instruments, identification labels and batch documentation for the trackability of the procedures. In this way, the closed, sterile and sealed containers can be stored ready for use in quantity and type commensurate to the workloads of the operating room for surgical activity both programmed and in emergency. After the use of said instruments, the same are picked up in the container, pre-treated inside this latter with disinfecting solutions, closed with a cover for evacuation and collected in special trolleys for transport to the sterilization facility for the execution of decontamination, washing, and thermo-chemical disinfection. The instruments are checked and subject to possible maintenance or substitution where necessary, repackaged into ordered sets in said special metal racks, closed in the container and subjected to sterilization in autoclave with consequent repetition of all the preceding steps.

Further characteristics and advantages of the present method will mostly become evident from the detailed description of a form of preferred - but not exclusive - execution of a method for arrangement of sterilized procedural set of surgical instruments for service to operating rooms with cyclical and traceable treatment of sanitizing, maintenance, sterilization and movement.

### Brief Description Of the Drawings

The attached figures refer to a particular embodiment of the method and of the structure comprising suitable environments and equipment for a preferred but not exclusive execution thereof.

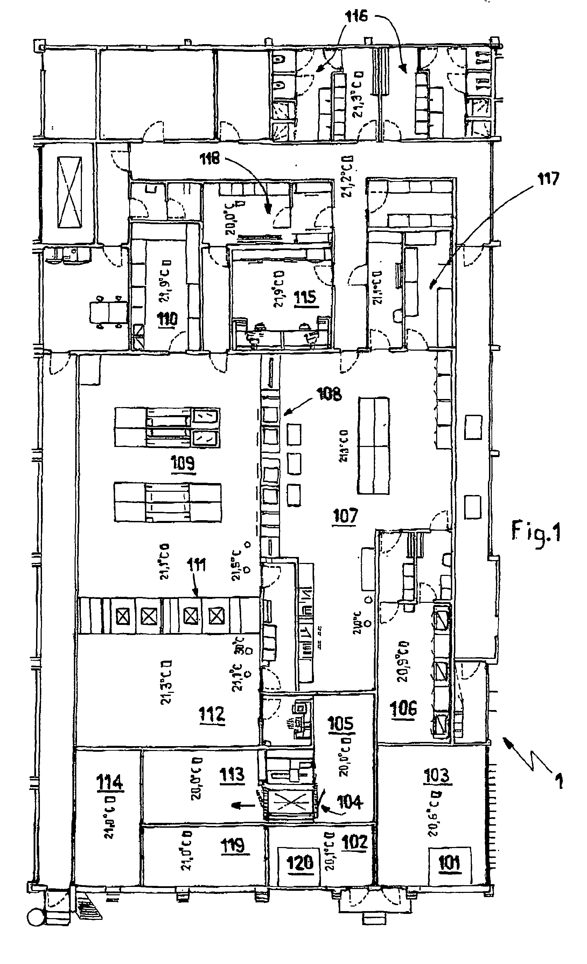

- Figure 1 is a plan view of the environment which is part of the patented structure in which the operations of sanitizing, disinfection, packaging and sterilization of surgical instruments for service in operating rooms are carried out;

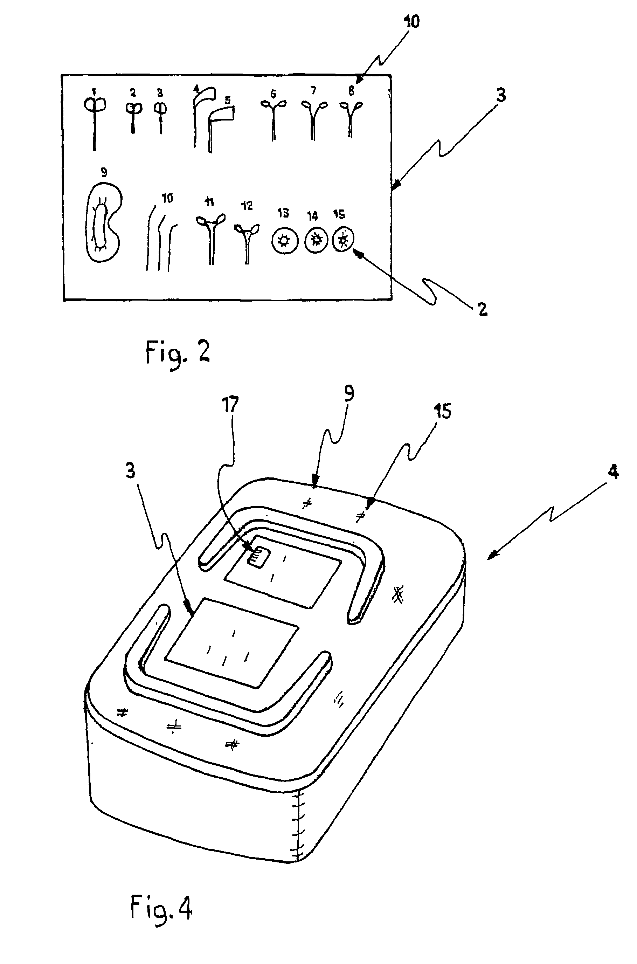

- Figure 2 is a graphic reproduction of a set of surgical instruments-,

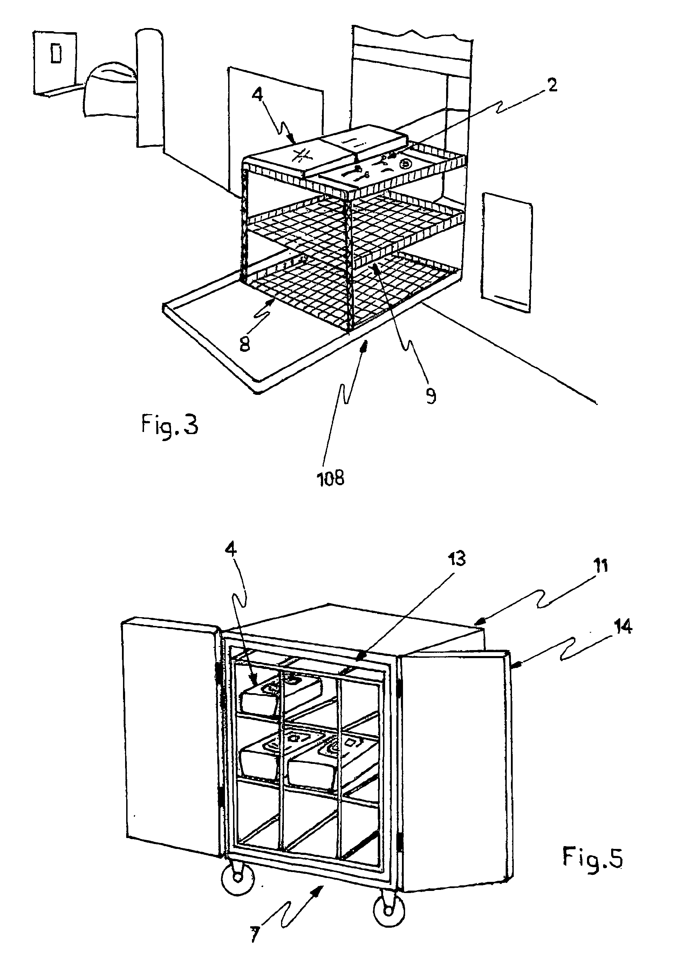

- Figure 3 is a perspective view of a surgical instrument washing machine
- Figure 4 is a perspective view of a container of surgical instruments, closed and comprising graphic means for the identification of the contents;
- Figure 5 is a perspective view of a collection trolley for closed containers;

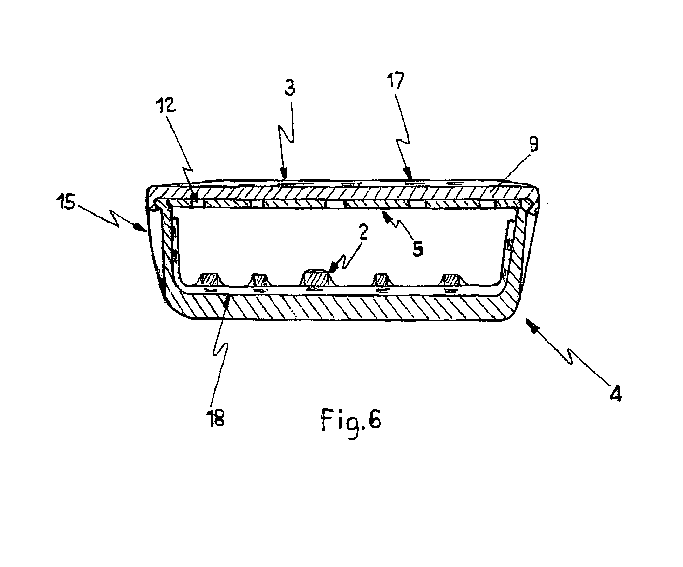

- Figure 6 is a view in section of the container of figure 4.

### Detailed Description Of A Particular Embodiment Of the invention

The method will now be described following the steps that are carried out inside suitable environments of the structure, making reference to the Figures described above.

With particular reference to such Figures, the numeral 1 indicates the whole set of environments in which are carried out the various steps of the operations of sanitizing, disinfection, packaging and sterilization of surgical instruments for service to operating rooms, said environments being preferably part of a service structure separated from the hospital or hospitals served.

The term "structure" used here and hereinafter shall refer to the entire set of environments for sanitizing, maintenance, disinfection, packaging and sterilization.

Furthermore, although a particular layout of the environments dedicated to the various steps of the operations of sanitizing, disinfection, packaging and sterilization of surgical instruments for service to operating rooms, is herein described, as will be clear to the skilled person, such a layout cannot be considered limiting to the present method; also not intended to be limiting to the method are the forms, the dimensions and the layout of the environments, the layout of the various apparatus set in the environments themselves and which serve for the operations of sanitizing, disinfection, packaging and sterilization of surgical instruments for service to operating rooms; also not limiting to the method is the type of equipment used for the execution of the said operations.

Step a) of the method which is the subject of the present invention relates to the identification of the set of surgical instruments 2 most suitable for the execution of each particular surgery and its graphic reproduction 3. Said identification is carried out, for instance, on the basis of the type of the surgery and usually in agreement with the surgeon who performs the surgery, with consequent choice of the quality, type and number of the surgical instruments 2 to be adopted.

Once the set of surgical instruments 2 is identified, one proceeds to the choice of the layout of the instruments inside the container in which these are set aside, of the support racks 18 on which said instruments 2 are supported, and of the container 4 or of the series of containers, substantially parallelepiped in shape, most suited to receive said rack 18 or said racks and said surgical instruments 2.

The graphic reproduction 3 of the distribution of said surgical instruments 2 on every support rack 18 is produced, for instance, by a photographic device, with the additional insertion of identification numbers 10 of the relative position to every surgical tool 2.

Step b) of the method which is the subject of the present invention relates to the arrangement of one or more sets of surgical instruments 2 for every surgery and, where necessary, for every surgeon who will carry out the operation, selected on the basis defined in the aforesaid step a), the set being sterilized and orderly placed in a related container 4, which is closed with a sterilization cover 5 having passages 12 for the sterilization steam, with a second means of coverage 9 for closing the container 4 during evacuation, and with a plastic film 15 (for instance, a thermo-shrink plastic film), for airtight sealing of the closed container 4. Between said closed container 4 and said plastic film 15 is placed the graphic reproduction 3 of the set of surgical instruments contained therein. Together with the graphic reproduction 3 is preferably placed a further means of identification of the set of surgical instruments 2, such as, for instance, a bar code 17 and/or a further identification code such as a color code (here not shown) varying according to the discipline or to the surgery type of the set, rather than an alphanumeric denomination. Said means of sterilization cover 5 and evacuation cover 9 are fitted to the container 4 by, for instance, a restraining, interference or snap-lock means of fixture. Said surgical instruments 2 are positioned inside the container 4 supported on one or more support racks 18 suitably made for hosting a set of surgical instruments 2 for each surgery and for each surgeon.

Step c) of the method object of the present invention relates to the storage of the closed containers 4 in a suitable room, preferably, but not exclusively, in proximity of the operating room of the hospital in which said surgical instruments 2 are used for that particular surgery and for every surgeon destined to carry out the operation. The transport of said closed container 4 to the room of storage, preferably, but not exclusively, in proximity of the operating room of the hospital is effected with the aid of a means of transport of the trolley 7 type, comprising a first external hollow body 11 substantially parallelepiped in shape with airtight edges 13 and a second inside body comprising a series of rods connected such a way as to provide a shelf for supporting a plurality of said containers 4.

Said collection trolley 7 is previously sanitized and it is provided with doors 14 for airtight closure in correspondence to its edges.

Said step c) can be preceded, according to a preferred - but not exclusive - embodiment of this method, by an operation of transport in the hospital department by a vehicle suitably designed to receive at least one collection trolley 7, each trolley containing at least one closed container 4, to guarantee maximum stability during transport.

Step d) of the method object of the invention relates to the collection of the closed container 4 from the storeroom of the operating room, on the basis of a programmed or emergency surgery, transport into the operating room, the opening of said container 4, by removing, in sequence, the plastic film 15, the means of coverage for evacuation 9 and the first means of coverage for sterilization 5. The support racks 18 containing the surgical instruments 2 are then extracted from the container 4 for the preparation of the operating table, having previously seen the graphic reproduction 3 for the execution of the surgery.

Step e) of the method object of the invention relates to the collection of the surgical instruments 2 used by the surgeon for the surgery, their re-insertion inside the container 4 and the closing of said container 4 with the apposition of the second means of coverage for evacuation 9, bound to the container 4 by, for instance, a restraining, interference or snap-lock means of fixture.

During said step e), before closing the container 4, a pre-treatment operation is carried out with biocidal solution for decontamination of the used surgical instruments 2, which are then sprinkled with substances which stop or slow the development of bacteria, such as, for instance, surfactant bacterostatic gel. Subsequently, said closed container 4 is put inside a collection trolley 7 for movement in safety toward the structure in which the operations of sanitizing, disinfection, maintenance, packaging and sterilization of the used surgical instruments 2, of the related rack 18 and of the related container 4 are carried out. Said collection trolley 7 can internally house a multiplicity of containers 4, each container 4 can contain a related set of surgical instruments 2 and related support rack 18.

Step f) of the method which is the subject of the present invention relates to the treatment of washing and decontamination of the used surgical instruments 2 and of the related container 4. According to a preferred, but not exclusive, embodiment of this method, said step f) can be preceded by a transport operation in a structure specifically arranged to receive said trolley and to carry out the operations of said step f). Said transport operation is suitably carried out by vehicles designed to receive at least one collection trolley 7 - each trolley containing at least one closed container 4 - and to guarantee its maximum stability during the transport.

Said collection trolley 7, containing said container 4, is transferred to a first acceptance area 101 of the structure 1 housing the environments in which the various steps of treatment of washing and decontamination of the surgical instruments 2, of the support racks 18, of the containers 4, and of the collection trolleys 7 are carried out. Said structure 1 is specifically arranged for receiving said collection trolley 7, said containers 4 and said surgical instruments 2, since it is endowed with means for the independent regulation and control of the temperature, pressure and humidity, inside every environment.

Advantageously, said structure 1 has further environments 116, 117 and 118 for dressing of the employed personnel who carries out the requested operations, and for monitoring 115 the progress of the work for the various steps in each environment and for every container 4 entering, due to the presence of a means of automatic recognition of the sets of surgical instruments 2, of the containers 4 and of the support racks 18, such as, for instance, optical readers working on the passage of said set of surgical instruments, said containers and said racks, from one environment to another of the structure 1.

From the acceptance area 101 of said structure 1 the collection trolley 7 is moved toward the environment 105, passing through the environment 103, said environments 103 and 105 being temperature controlled and having pressure increasing from the environment 103 to the environment 105. Inside said environment 105, said containers 4 are withdrawn from said collection trolley 7 and transported toward the environment 106, where they are opened by the removal of the second means of air-tight coverage 9. Every open container 4 is emptied of the liquids resulting from the decontamination and together with its contents, comprising the set of surgical instruments 2 and the support racks 18, is subjected to a first pre-washing treatment. Said operation of pre-washing is performed preferably, but not exclusively, inside airtight containers provided with heating devices.

In the meantime, the collection trolley 7 is subjected to a treatment of sanitizing by passage through a treatment tunnel 104 in which, for instance, use is made of high pressure water-jets with detergents and disinfectants, and with a following biocide step with high-temperature steam, said trolley 7 subsequently being subjected to a drying cycle and finally transferred into the storage environment 113 for its reuse.

The surgical instruments 2, the containers 4 and the support racks 18, immediately after being subjected to the first pre-washing treatment, are transferred into environment 107 where they undergo a further manual washing treatment and decontamination, before being subjected to automatic washing operations. Said operations of manual washing and decontamination are carried out using solutions, such as, for instance, of a proteolytic enzyme type, and/or using a further step of treatment with ultrasounds. The preparation for the automatic washing is completed by the positioning of said surgical instruments 2, of said containers 4 and of said support racks 18 on special washing racks 8. At the end of step f) said surgical instruments 2 are checked and counted.

Said environment 107 is at a higher pressure than the environments 105 and 106.

Step g) of the method object of the present invention relates to the treatment of thermo-chemo-disinfection of the used surgical instruments 2, of the related container 4 and of the support racks 18. Said step is preferably, but not exclusively, carried out by automatic treatment in a washing device, such as, for instance, surgical instrument washer apparatuses 108, which are capable of submitting said instruments 2, said containers 4 and said support racks to specific cycles of treatment. At the end of the loading of said surgical instrument washer apparatuses by the positioning of the washing racks 8 in special baskets of every surgical instrument washer apparatus 108, said instruments 2, said containers 4 and said support racks 18 are subjected to washing cycles that can comprise, in particular, but not exclusively, one or more of the following operations:
washing
treatment with detergents
treatment with steam
lubricating treatment.

Said washing operation is carried out with substances, such as, for instance, tensioactive agents added to high pressure water jets.

Said treatment with detergents is carried out with substances, such as, for instance, enzymatic detergents.

Said lubricating treatment is carried out with substances, such as, for instance, emulsions of synthetic medical oils.

At the end of the cycle of automatic treatment, the surgical instruments 2, the containers 4 and the support racks 18 are subjected to drying while still inserted into the surgical instrument washer apparatus 108.

Step h) of the method object of the invention relates to the repackaging of the surgical instruments 2 supported on their related support racks 18 inside the related container 4. Said step h) is carried out inside the environment 109, which is subjected to a further control in order to reduce contaminants in the air and has a higher internal pressure than environment 107. Preferably, said surgical instrument washers are positioned in correspondence of the passage between the environment 107 and the environment 109, so as to constitute a further barrier for the progress of polluting substances from environment 107 to environment 109.

The operation of re-packaging is facilitated by the presence of the graphic reproduction 3 of the set of surgical instruments 2 subjected to treatment and from the presence of a bar code identification that allows to recall said graphic reproduction 3 as soon as the container 2 has passed through a mean of automatic recognition of the type constituted, for instance, by a optic reader.

The operation of repackaging the surgical instruments can be carried out, preferably, but not exclusively, on worktables of a plastic non-reflecting material to give a better visualization of the surgical instruments, and on work-surfaces having a certain plasticity such as to avoid damaging the same instruments 2 during their manipulation.

Inside the environment 109 said surgical instruments contents inside the related containers 4, preferably, but exclusively, can undergo a non-functionality check, and, where necessary, be replaced if worn or damaged.

Said damaged or worn instruments are, where necessary, replaced with new sanitized instruments stored near one or more storage environments 110.

Step i) of the method object of the invention relates to the application of the related means of coverage 5 on the container 4 containing the related set of surgical instruments 2 placed on the related racks 18 and following insertion of said closed containers 4 inside a suitable sterilization device, such as, for instance, an autoclave 111, with use of movement trolleys suitably constructed to house said containers 4 and to enter inside said autoclaves. Preferably, said autoclaves 111 are positioned in correspondence of the passage between environment 109 and environment 112, thus constituting, further barriers to the passage of polluting substances from environment 109 to environment 112. Said environment 112 it is kept at a higher air pressure than the environment 109.

The activation of the autoclaves 111 enables the sterilization of the containers 4, the surgical instruments 2 contained therein and of the related support racks 18 for said surgical instruments 2, due to the fact that the sterilization steam passes through the passages 12 made on the cover 5 of the container 4. The above sterilization operation is also carried out contemporarily on at least one test-tube to verify the functioning of the autoclave and can be preceded and followed by weighing the packaged container, using high precision weighing devices. Said weighing operation permits verification that, subsequent to the sterilization, there is no unsuitable condensate present inside the container 4.

At the end of the sterilization operation, the containers 4, the surgical instruments 2 and the related support racks 18 are taken from the autoclaves 111 to the side of the environment 112.

Step I) of the method which is the subject of the present invention is carried out inside the sterile environment 112 and relates to the application of a second means of coverage 9 that it is locked onto said container 4 by a means of fixture such as, for instance, restraining or interference or snap-lock means of fixture, and to the positioning, on the top of said second means of coverage 9, of the elements of identification of the container 4, such as, for instance, a graphic reproduction 3 of the set of surgical instruments 2 and a systems of identification with bar codes 17.

Step m) of the method object of the present invention relates to the sealing of the container in the sterile environment 112, through the application of a plastic film 15, preferably, but not exclusively, of the thermo-shrink type, that shrouds the closed container 4, and that, once heated, it is withdrawn guaranteeing a further airtight seal inside the closed container 4, the protection of the container and a further system to prevent any accidental opening of the container.

During said step m) the container 4, which comprises a first means of coverage 5, a second means of coverage 9, a means of graphic identification 3 and a plastic film 15, is moved to environment 114 and repositioned on the collection trolleys 7, previously sanitized and moved from environment 113 to environment 114.

Said environment 114 is kept at a higher pressure than environments 112 and 113.

Step n) of the method object of the present invention relates to the transport of the collection trolleys 7 from the environment 114 to the environment 119, where they wait for their loading on the suitable vehicles and their transport to the hospital compartment, where the containers 4 are stored. The loading of said collection trolleys 7 on said vehicles happen in the loading zone 120 passing through the environment 102 of the structure 1.

It has been ascertained in practice that the method herein described, which is the subject matter of the present invention, achieves the purposes proposed.

In fact, said structure 1 is so arranged that the pressure of the air in every environment is lower than the pressure of the air in the following environment involving ever smaller degrees of contaminating substances, as one draws near to the sterilization step, in the environment 111, and of closing of the containers sterilized with further fitting of the plastic film 15 in the sterile environment 112.

Subsequently, advantageous is the layout of the various environments that, according to the method object of the present invention, guarantees optimal anti-bacteria protection of the surgical instruments due to the fact that from step d) to the step n) the operations carried out involve adjacent and connected environments through airtight passages or apparatuses with double openings.

It should be noticed that the operations that are carried out in environments 102, 103, 104, 105, 106, 107, 109, 112, 113, 114 and 119 present inside structure 1, are performed in advantageous way subsequently by personal wearing anti-contamination garments and footwear to protect themselves in the environments 106 and 107 and to protect the environments in 109, 112, 113, 114. Furthermore, all the passages that conduct from one environment to another one are made airtight to prevent the transfer of contaminating substances from one environment to another and falls of pressure.

Furthermore, the method object of the patent, associating for every type of surgery and, where necessary, for every surgeon a unique kit comprising container, surgical instruments and support racks, endowed with means of identification with bar codes or other methods, permits complete trackability to be guaranteed for every procedural set, step by step, and environment by environment, during every treatment and/or operation present in the step.

Said trackability is managed by operators present inside a further environment 115 present inside the structure 1 who, with the aid of computerized means, coordinate and organize the various steps of the method and can, where necessary, track down any container, surgical tool or rack 18 during any step.

The method and the structure object of the present invention, thus conceived are capable of numerous changes and variations all falling within the inventive concept thereof.

Furthermore, all the details can be are replaced by others technically equivalent. In practice the materials employed, the type of substances used in the different operations that characterize the various steps and the apparatuses used for the execution of the operations can be any according to the demands.

## Claims

1. Method for the arrangement and treatment of sanitizing, disinfection maintenance, packaging and sterilization of surgical instruments for service to operating rooms, comprising the following steps:
a) identification of a set of surgical instruments demanded for every type of surgery and graphic reproduction of said set;
b) arrangement of one or more sets of surgical instruments for every surgery, every set being sterilized and orderly prepared in a related closed container carrying the graphic reproduction of the instruments contained therin and a means of identification of the same;
c) storage of the closed containers;
d) withdrawing and opening of the container containing the set of instruments destined for a programmed operation and use of said instruments;
e) collection of the used instruments in the container and closing of the same container;
f) treatment of washing and decontamination of the used instruments and the related container;
g) treatment of thermo-chemical disinfection of the used instruments and of the related container;
h) repackaging of the instruments in the container with checking of the same instruments;
i) application to the container of a cover for sterilization and sterilization treatment in autoclave of this latter and of the contained instruments;
l) application to the container of a cover for evacuation, and of elements for the identification of the same container and of the related content;
m) airtight closing of the container;
n) transport to the store or stores of step c);
o) repetition of the steps from d) to n) for every operation in which the instruments of the container are used.

2. Method according to the claim 1. **characterized by** steps a) and b) of identification and arrangement of the set being carried out for every type of operation and for every surgeon destined to carry out the operation.

3. Method according to claim 1 or 2, **characterized by** there being a step of pretreatment with biocidal solution and decontamination of the used instruments collected in the container during step e).

4. Method according to any of the preceding claims, **characterized by** the steps by f) to m) being carried out in a specifically arranged structure to which the used containers are transported and from which the sterile containers are transported for storage.

5. Method according to the claim 4, **characterized by** the transport of the containers being carried out after the instruments have been arranged in a collection trolley, which in its turn is sanitized before transport of the containers to the store or stores.

6. Method according to any of the preceding claims, **characterized by** the treatment of step f) comprising the opening of the container, the disposal of any liquids remaining in it and operations of pre- washing of the instruments.

7. Method according to the claim 6 **characterized by** the treatment of step f) comprising furthermore a treatment with ultrasounds.

8. Method according to one of the preceding claims, **characterized by** step f) comprising counting and checking of the instruments of the set.

9. Method according to one of the preceding claims, **characterized by** step g) being carried out in suitable surgical instrument washer apparatuses to submit the instruments to specific cycles of treatment.

10. Method according to the claim 9, **characterized by** the treatment carried out in the surgical instrument washer apparatus including one or more of the following operations:
washing
treatment with detergents
treatment with steam
lubricating treatment.

11. Method according to one or more of the preceding claims, **characterized by** the packaging of step h) being carried out following checking and possible substitution of one or more worn or damaged instruments.

12. Method according to one or more than the preceding claims, in which the surgical instruments are packaged in the containers on support racks, **characterized by** the support racks being subjected to the steps of treatment by f) to i).

13. Method according to any of the preceding claims, **characterized by** step i) of treatment being contemporarily carried out on a test- tube.

14. Method according to any of the preceding claims, **characterized by** the packaged container being weighed before and after the step i) of sterilization.

15. Method according to one or more than the preceding claims, **characterized by** the instrument set and the container being traceable in every step of treatment.

## Patentansprüche

1. Verfahren zur Anordnung und Behandlung zur Keimtötung, Desinfektion, Wartung, Verpackung und Sterilisation chirurgischer Instrumente für den Einsatz in Operationssälen, umfassend die folgenden Schritte:
a) Identifizierung eines Satzes chirurgischer Instrumente, welche für jede Art von Operation erforderlich sind sowie grafische Reproduktion dieses Satzes;
b) Anordnen eines oder mehrerer Sätze chirurgischer Instrumente für jede Operation, wobei jeder Satz sterilisiert und in einem zugehörigen verschlossenen Behälter geordnet vorbereitet ist, welcher die grafische Reproduktion der darin enthaltenen Instrumente trägt sowie ein Hilfsmittel zur Kennzeichnung derselben;
c) Lagerung der verschlossenen Behälter;
d) Entnahme und Öffnen des Behälters, welcher den Satz von Instrumenten enthält, welche für eine vorgesehene Operation bestimmt sind, sowie die Verwendung dieser Instrumente;
e) Sammeln der gebrauchten Instrumente in dem Behälter und Verschließen ebendieses Behälters;
f) Behandlung durch Spülen und Dekontaminieren der gebrauchten Instrumente und des zugehörigen Behälters;
g) Behandlung durch thermo-chemische Desinfektion der gebrauchten Instrumente und des zugehörigen Behälters;
h) erneutes Verpacken der Instrumente in den Behälter mit Überprüfung selbiger Instrumente;
i) Aufbringen einer Abdeckung auf den Behälter für die Sterilisation und eine sterilisierende Behandlung des letzteren und der enthaltenen Instrumente in einem Autoklaven;
l) Aufbringen einer Abdeckung auf den Behälter für die Evakuierung sowie von Elementen zur Kennzeichnung ebendieses Behälters und des zugehörigen Inhalts;
m) luftdichtes Verschließen des Behälters;
n) Abtransport zum Lager oder zu den Lagern von Schritt c);
o) Wiederholung der Schritte von d) bis n) für jede Operation, bei welcher die Instrumente des Behälters verwendet werden.

2. Verfahren nach Anspruch 1, **dadurch gekennzeichnet, dass** die Schritte a) und b) der Identifizierung und Anordnung des Satzes ausgeführt werden für jede Art von Operation und für jeden Chirurgen, welcher dazu bestimmt ist, die Operation durchzuführen.

3. Verfahren nach Anspruch 1 oder 2, **dadurch gekennzeichnet, dass** ein Schritt der Vorbehandlung mit biozider Lösung und Dekontamination der gebrauchten Instrumente, welche in dem Behälter während des Schritts e) gesammelt werden, existiert.

4. Verfahren nach einem der vorhergehenden Ansprüche, **dadurch gekennzeichnet, dass** die Schritte f) bis m) in einer speziell aufgebauten Struktur erfolgen, zu welcher die gebrauchten Behälter transportiert werden und von welcher die sterilen Behälter zur Lagerung abtransportiert werden.

5. Verfahren nach Anspruch 4, **dadurch gekennzeichnet, dass** der Abtransport der Behälter ausgeführt wird, nachdem die Instrumente in einem Sammelwagen angeordnet wurden, welcher seinerseits vor dem Abtransport der Behälter zum Lager oder zu den Lagern keimfrei gemacht wurde.

6. Verfahren nach einem der vorhergehenden Ansprüche, **dadurch gekennzeichnet, dass** die Behandlung in Schritt f) das Öffnen des Behälters, die Entsorgung jedweder darin verbliebener Flüssigkeiten sowie Arbeitsabläufe zum Vorspülen der Instrumente umfasst.

7. Verfahren nach Anspruch 6, **dadurch gekennzeichnet, dass** die Behandlung in Schritt f) darüber hinaus eine Behandlung mit Ultraschall umfasst.

8. Verfahren nach einem der vorhergehenden Ansprüche, **dadurch gekennzeichnet, dass** Schritt f) das Zählen und Überprüfen der Instrumente des Satzes umfasst.

9. Verfahren nach einem der vorhergehenden Ansprüche, **dadurch gekennzeichnet, dass** Schritt g) in geeigneten Spülvorrichtungen für chirurgische Instrumente durchgeführt wird, um die Instrumente speziellen Behandlungszyklen zu unterwerfen.

10. Verfahren nach Anspruch 9, **dadurch gekennzeichnet, dass** die Behandlung, welche in der Spülvorrichtung für chirurgische Instrumente erfolgt, einen oder mehrere der folgenden Arbeitsabläufe einschließt:
Spülen
Behandlung mit Detergenzien
Behandlung mit Dampf
Behandlung mit Schmiermittel.

11. Verfahren nach einem oder mehreren der vorhergehenden Ansprüche, **dadurch gekennzeichnet, dass** das Verpacken in Schritt h) ausgeführt wird in der Folge einer Überprüfung und möglichen Austausches eines oder mehrerer abgenutzter oder beschädigter Instrumente.

12. Verfahren nach einem oder mehreren der vorhergehenden Ansprüche, wobei die chirurgischen Instrumente in den Behältern auf Trägergestelle gepackt werden, **dadurch gekennzeichnet, dass** die Trägergestelle den Behandlungsschritten f) bis i) unterworfen werden.

13. Verfahren nach einem der vorhergehenden Ansprüche, **dadurch gekennzeichnet, dass** Schritt i) der Behandlung zeitweise an einem Reagenzglas durchgeführt wird.

14. Verfahren nach einem der vorhergehenden Ansprüche, **dadurch gekennzeichnet, dass** der gepackte Behälter vor und nach dem Schritt i) der Sterilisation gewogen wird.

15. Verfahren nach einem oder mehreren der vorhergehenden Ansprüche, **dadurch gekennzeichnet, dass** der Satz von Instrumenten und der Behälter in jedem Schritt der Behandlung nachverfolgbar ist.

## Revendications

1. Procédé de préparation d'un ensemble procédural d'instruments chirurgicaux stérilisés pour salles d'opération, avec traitement identifiable d'assainissement, d'entretien, d'emballage et de stérilisation, comprenant les étapes suivantes :
a) identification d'un ensemble d'instruments chirurgicaux requis pour chaque type d'intervention chirurgicale, et reproduction graphique de chaque ensemble ;
b) préparation d'un ou plusieurs ensembles d'instruments chirurgicaux pour chaque type d'intervention chirurgicale, chaque ensemble étant stérilisé et préparé de manière ordonnée dans un réceptacle en relation proche contenant la reproduction graphique des instruments contenus en lui, et moyen d'identification de ceux-ci ;
c) stockage des réceptacles fermes ;
d) extraction et ouverture du réceptacle contenant l'ensemble d'instruments destiné à une intervention programmée, et utilisation desdits instruments ;
e) collecte des instruments usagés à l'intérieur du réceptacle, et fermeture de ce même réceptacle ;
f) traitement de nettoyage et de décontamination des instruments usagés et du réceptacle associé ;
g) traitement de désinfection chimico-thermique des instruments usagés et du réceptacle associé ;
h) reconditionnement des instruments à l'intérieur du réceptacle, avec vérification de ces mêmes instruments ;
i) application, sur le réceptacle, d'un couvercle pour stérilisation, et traitement de stérilisation en autoclave de ce dernier et des instruments qu'il contient ;
l) application, sur le réceptacle, d'un couvercle pour évacuation, et d'éléments pour l'identification de ce même réceptacle et de son contenu associé ;
m) fermeture hermétique du réceptacle ;
n) transport vers le(s) emplacement(s) de stockage de l'étape c) ;
o) répétition des étapes d) à n) pour chaque intervention au cours de laquelle les instruments du réceptacle sont employés.

2. Procédé selon la revendication 1, **caractérisé par le fait que** les étapes a) et b) d'identification et de préparation de l'ensemble sont accomplies pour chaque type d'intervention et pour chaque chirurgien qui doit pratiquer l'intervention.

3. Procédé selon la revendication 1 ou 2, **caractérisé par** une étape de traitement préalable à l'aide d'une solution biocide et de décontamination des instruments usagés collectés à l'intérieur du réceptacle au cours de l'étape e).

4. Procédé selon l'une quelconque des revendications précédentes, **caractérisé par le fait que** les étapes f) à m) sont accomplies dans une structure spécifiquement prévue à cet effet, vers laquelle les réceptacles usagés sont transportés, et à partir de laquelle les réceptacles stériles sont transportés en vue du stockage.

5. Procédé selon la revendication 4, **caractérisé par le fait que** le transport des réceptacles est accompli après que les instruments ont été installés dans un chariot de collecte qui est à son tour désinfecté avant le transport des réceptacles vers le(s) emplacement(s) de stockage.

6. Procédé selon l'une quelconque des revendications précédentes, **caractérisé par le fait que** le traitement exécuté à l'étape f) comprend l'ouverture du réceptacle, la mise au rebut de tout liquide pouvant encore s'y trouver, ainsi que des opérations de nettoyage préalable des instruments.

7. Procédé selon la revendication 6, **caractérisé par le fait que** le traitement exécuté à l'étape f) comprend en outre un traitement aux ultrasons.

8. Procédé selon l'une quelconque des revendications précédentes, **caractérisé par le fait que** le traitement exécuté à l'étape f) comprend le comptage et la vérification des instruments de l'ensemble.

9. Procédé selon l'une des revendications précédentes, **caractérisé par le fait que** l'étape g) est accomplie dans des dispositifs de nettoyage d'instruments chirurgicaux adaptés de façon à soumettre les instruments à des cycles de traitement spécifiques.

10. Procédé selon la revendication 9, **caractérisé par le fait que** le traitement exécuté dans le dispositif de nettoyage d'instruments chirurgicaux comprend une ou plusieurs des opérations suivantes :
nettoyage ;
traitement à l'aide de détergents ;
traitement à la vapeur ;
opération de lubrification.

11. Procédé selon une ou plusieurs des revendications précédentes, **caractérisé par le fait que** le conditionnement réalisé à l'étape h) est exécuté à la suite de la vérification et d'un remplacement éventuel d'un ou plusieurs instruments usés ou endommagés.

12. Procédé selon une ou plusieurs des revendications précédentes, dans lequel les instruments chirurgicaux sont conditionnés dans des réceptacles placés sur des bâtis de support, le procédé étant **caractérisé par le fait que** les bâtis de support sont soumis aux étapes de traitement f) à i).

13. Procédé selon l'une des revendications précédentes, **caractérisé par le fait que** l'étape i) de traitement est accomplie en même temps sur un tube d'essai.

14. Procédé selon l'une des revendications précédentes, **caractérisé par le fait que** le réceptacle conditionné est pesé avant et après l'étape i) de stérilisation.

15. Procédé selon une ou plusieurs des revendications précédentes, **caractérisé par le fait que** l'ensemble d'instruments et le réceptacle peuvent être tracés à chaque étape du traitement.
